(19) Europäisches Patentamt
European Patent Office
Office européen des brevets

(11) Veröffentlichungsnummer: **0 016 371**
**A1**

(12) # EUROPÄISCHE PATENTANMELDUNG

(21) Anmeldenummer: 80101097.6

(22) Anmeldetag: 05.03.80

(51) Int. Cl.³: C 07 D 409/12
C 07 D 333/36, A 61 K 31/38
A 61 K 31/415

(30) Priorität: 14.03.79 CH 2415/79
10.01.80 CH 171 80

(43) Veröffentlichungstag der Anmeldung:
01.10.80 Patentblatt 80/20

(84) Benannte Vertragsstaaten:
AT BE CH DE FR GB IT LU NL SE

(71) Anmelder: F.HOFFMANN-LA ROCHE & CO.
Aktiengesellschaft

CH-4002 Basel(CH)

(72) Erfinder: Hunkeler, Walter, Dr.
Im Stigler 32
CH-4465 Magden(CH)

(72) Erfinder: Kyburz, Emilio, Dr.
Unterer Rebbergweg 127
CH-4153 Reinach(CH)

(74) Vertreter: Kellenberger, Marcus, Dr. et al,
Grenzacherstrasse 124 Postfach 601
CH-4002 Basel(CH)

(54) **Neue Harnstoffderivate, Verfahren und Zwischenprodukte zu deren Herstellung und Arzneimittel enthaltend ein solches Harnstoffderivat sowie die Verwendung eines solchen Harnstoffderivates bei der Bekämpfung von Krankheiten.**

(57) Die Harnstoffderivate der nachstehenden Formel

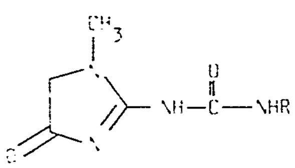

worin R 2-Thienyl, 3-Thienyl oder 5-Halogen-3-thienyl bedeutet,
weisen bei einer sehr geringen Toxizität eine selektive anxiolytische Wirkung auf, ohne dass sie die bei Anxiolytica normalerweise vorhandenen muskelrelaxierenden und sedierenden Eigenschaften besitzen, und eignen sich demnach für die Behandlung oder Verhütung von Angstzuständen. Sie können erhalten werden, indem man Kreatinin mit einem entsprechenden Isocyanatothiophen umsetzt.

EP 0 016 371 A1

F. Hoffmann-La Roche & Co. Aktiengesellschaft, Basel, Schweiz

RAN 4008/300

Neue Harnstoffderivate, Verfahren und Zwischenprodukte zu deren Herstellung und Arzneimittel enthaltend ein solches Harnstoffderivat sowie die Verwendung eines solchen Harnstoffderivates bei der Bekämpfung von Krankheiten.

Die vorliegende Erfindung betrifft neue Harnstoffderivate. Im speziellen betrifft sie 1-(1-Methyl-4-oxo-2-imidazolin-2-yl)-3-thienyl-harnstoffe der allgemeinen Formel

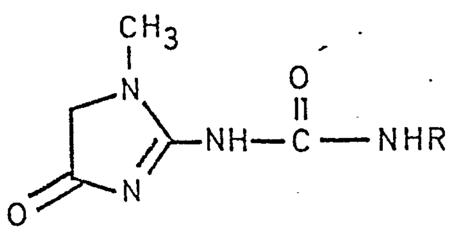

I

worin R 2-Thienyl, 3-Thienyl oder 5-Halogen-3-thienyl bedeutet.

Kbr/3.1.80

Die Verbindungen der obigen Formel I können auch in der tautomeren Form der allgemeinen Formel

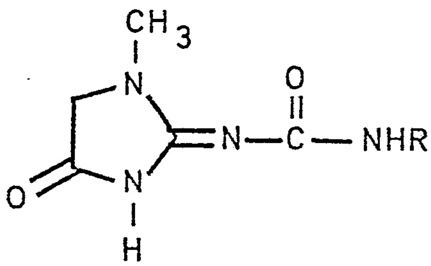

Ia

worin R die oben abgegebene Bedeutung besitzt, existieren.

Der in dieser Beschreibung verwendete Ausdruck "Halogen" umfasst die vier Halogene Fluor, Chlor, Brom und Jod, wobei Chlor und Brom bevorzugt sind; besonders bevorzugt ist Chlor.

Gegenstand der vorliegenden Erfindung sind die Harnstoffderivate der obigen Formel I, deren Herstellung und Zwischenprodukte zu deren Herstellung, ferner Arzneimittel, enthaltend ein Harnstoffderivat der obigen Formel I und die Herstellung solcher Arzneimittel sowie die Verwendung eines Harnstoffderivates der obigen Formel I bei der Bekämpfung bzw. Verhütung von Krankheiten.

Die Harnstoffderivate der obigen Formel I können erfindungsgemäss dadurch hergestellt werden, dass man Kreatinin der Formel

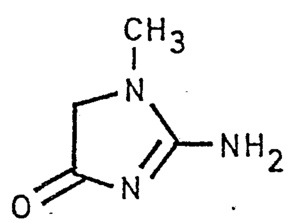

II

mit einem Isocyanatothiophen der allgemeinen Formel

OCN-R          III

worin R die oben angegebene Bedeutung besitzt, umsetzt.

Kreatinin der obigen Formel II wird nach an sich bekannten Methoden mit einem Isocyanatothiophen der obigen Formel III umgesetzt. Bei dieser Umsetzung arbeitet man zweckmässigerweise mit etwa äquimolaren Mengen der beiden Ausgangsstoffe. Die Umsetzung wird vorzugsweise in einem wasserfreien inerten aprotischen polaren organischen Lösungsmittel durchgeführt. Beispiele geeigneter Lösungsmittel sind Aether, wie Tetrahydrofuran oder Dioxan, Dimethylformamid, Dimethylsulfoxid, Hexamethylphosphorsäuretriamid und dergleichen. Nach beendeter Umsetzung wird das Produkt durch Zusatz von kaltem Wasser zum Reaktionsgemisch ausgefällt. Das Rohprodukt kann dann durch Umkristallisation aus einem Lösungsmittel gereinigt werden.

Beim als Ausgangsmaterial verwendeten Kreatinin der obigen Formel II handelt es sich um eine bekannte Verbindung. Von den unter die obige Formel III fallenden Verbindungen ist das 2-Isocyanatothiophen bekannt, während die übrigen Verbindungen noch neu und ebenfalls Gegenstand der vorliegenden Erfindung sind. Die noch neuen 3-Isocyanatothiophene können in zur Herstellung des bekannten 2-Isocyanatothiophens analoger Weise hergestellt werden, zweckmässig ausgehend von einer Thiophen-3-carbonsäure, welche in an sich bekannter Weise in ein reaktionsfähiges Derivat, z.B. das entsprechende Säurechlorid, übergeführt wird, welches ebenfalls in an sich bekannter Weise durch Umsetzen mit Natriumazid und anschliessendem Erhitzen in das erwünschte 3-Isocyanatothiophen übergeführt wird, wobei die als Zwischenprodukte auftretenden reaktionsfähigen Derivate, z.B. die Säurechloride, und Säureazide nicht isoliert werden müssen. Auch das 3-Isocyanatothiophen braucht vor dessen Ueberführung in das entsprechende Harnstoffderivat der obigen Formel I nicht isoliert zu werden. Bei den Thiophen-3-carbonsäuren handelt es sich um bekannte Verbindungen.

Die Harnstoffderivate der obigen Formel I sind wertvolle Arzneimittel und können insbesondere als Anxiolytika verwendet werden. Die Verbindungen der obigen Formel I weisen nur eine sehr geringe Toxizität auf, und es hat sich gezeigt, dass sie eine sehr selektive anxiolytische Wirkung besitzen, ohne dass sie die bei Anxiolytika normalerweise vorhandenen muskelrelaxierenden und sedierenden Eigenschaften besitzen. Die anxiolytische Wirkung wurde im nachstehend beschriebenen Tierversuch experimentell nachgewiesen.

Die Testapparatur ist eine Eintasten-Skinnerbox mit Futterpillengeber.

Während 3 einstündigen Vorversuchen (an drei verschiedenen Tagen) werden hungrige weibliche Füllinsdorfer Ratten (SPF, 190-230 g) darauf trainiert, die Taste des Futterpillengebers zu drücken, um Futterpillen von je 45 mg zu erhalten (jeder Tastendruck wird belohnt); im dritten Vorversuch erreichen die Ratten eine Rate von 150-200 Tastenbetätigungen pro Stunde.

BAD ORIGINAL

In einem vierten Vorversuch wird jede durch Tastendruck hervorgerufene Pillenbelohnung mit einem kurzen elektrischen Fuss-Schock (1,0 mA) kombiniert. Die Ratten, welche mit dieser Konflikt-Situation konfrontiert sind, betätigen anfangs noch etwa 5-10mal die Drucktaste und hören dann aus Angst gänzlich auf.

In einem fünften Vorversuch können die Ratten die Taste des Futterpillengebers wieder ohne zusätzlichen Fuss-Schock drücken, wobei wiederum eine Rate von 150-200 Tastenbetätigungen pro Stunde erreicht wird.

In einem sechsten Vorversuch wird eine Selektion der Testtiere durchgeführt. Den Testtieren werden eine halbe Stunde vor Beginn dieses Vorversuches peroral 10 mg/kg Chlordiazepoxid verabreicht; jede Pillenbelohnung wird wiederum mit einem Fuss-Schock kombiniert (Konflikt). Nur Ratten, die in diesem Vorversuch eine Rate von 20-50 Tastenbetätigungen erreichen (vgl. die 5-10 Tastenbetätigungen im vierten Vorversuch) werden als geeignete Testtiere für die Prüfung von potentiellen Anxiolytika behalten. Die Eliminationsrate in diesem Vorversuch beträgt 5%.

Im Hauptversuch werden zur Prüfung der potentiellen Anxiolytika pro Substanz und pro Dosis in der Regel 8 Ratten eingesetzt. Eine unbehandelte Kontrollgruppe ist nicht erforderlich, da jedes Tier als seine eigene Kontrolle dient. Die Testsubstanzen, welche in einem Gemisch von destilliertem Wasser (10 ml) und Tween 80 (2 Tropfen) gelöst oder suspendiert sind, werden den Tieren mit Hilfe einer Schlundsonde eine halbe Stunde vor dem einstündigen Hauptversuch verabreicht. Während des Hauptversuchs, in welchem bei jedem Tastendruck die Pillenbelohnung mit einem Fuss-Schock kombiniert ist (Konflikt), wird die Rate der Tastenbetätigungen pro Stunde registriert.

Die erste signifikant anxiolytisch wirksame Dosis wird mit dem Wilcoxon-Test (Vergleich von Paaren) bestimmt, indem die Anzahl der Tastenbetätigungen im Hauptversuch (Fuss-Schock, nach Vorbehandlung durch Testsubstanz) mit der Anzahl der Tastenbetätigungen im Kontrollversuch (Fuss-Schock, nach Vorbehandlung mit Kochsalzlösung) direkt verglichen wird.

Im vorstehend beschriebenen Versuch beträgt die erste signifikant anxiolytisch wirksame Dosis für den 1-(5-Chlor-3-thienyl)-3-(1-methyl-4-oxo-2-imidazolin-2-yl)harnstoff, den 1-(1-Methyl-4-oxo-2-imidazolin-2-yl)-3-(3-thienyl)harnstoff bzw. den 1-(1-Methyl-4-oxo-2-imidazolin-2-yl)-3-(2-thienyl)harnstoff 3 mg/kg, 10 mg/kg bzw. 30 mg/kg.

Hingegen zeigen die beiden unsubstituierten Verbindungen im maximalen und minimalen Elektroschock, im Versuch am Rotierstab, beim Kamintest, beim Antipentetrazoltest und beim 3-Mercaptopropionsäure-Test (jeweils an der Maus) bis zu einer Dosis von 300 mg/kg keinerlei Aktivität; die 5-Chlorverbindung zeigt im maximalen Elektroschock eine $ED_{50}$ von $>$ 100 mg/kg und beim 3-Mercaptopropionsäure-Test eine $ED_{50}$ von 76 mg/kg, während diese Verbindung im Versuch am Rotierstab, beim Kamintest und beim Antipentetrazoltest ebenfalls bis zu einer Dosis von 300 mg/kg keinerlei Aktivität besitzt. Bei den oben genannten Versuchen handelt es sich um allgemein bekannte Standardmethoden zur Bestimmung von sedierender und muskelrelaxierender Wirkung.

Die Harnstoffderivate der obigen Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Harnstoffderivate der obigen Formel I mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc..

Zur Herstellung von Lösungen und Sirupen eignen sich als Excipientien z.B. Wasser, Polyole, Sacharose, Invertzucker, Glucose und dergleichen.

Für Injektionslösungen eignen sich als Excipientien z.B. Wasser, Alkohole, Polyole, Glycerin, vegetabile Oele etc..

Für Suppositorien eignen sich als Excipientien z.B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxydantien enthalten. Sie können auch noch andere therapeutisch wertvolle Stoffe enthalten.

Eine geeignete pharmazeutische Dosierungseinheit kann etwa 0,5-50 mg, vorzugsweise 1-30 mg, einer Verbindung der obigen Formel I enthalten. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 0,01 mg/kg bis etwa 2 mg/kg angemessen sein. Diese Dosen sind jedoch lediglich im Sinne von Beispielen aufzufassen; die spezifische Dosierung muss in jedem Fall den individuellen Bedürfnissen angepasst werden.

In den nachfolgenden Beispielen, welche die vorliegende Erfindung illustrieren, ihren Umfang jedoch in keiner Weise einschränken sollen, sind sämtliche Temperaturen in Celsius-Graden angegeben.

Beispiel 1

Eine Suspension von 3,20 g (25,6 mMol) 2-Isocyanatothiophen und 2,90 g (25,6 mMol) Kreatinin in 25 ml wasserfreiem Dimethylformamid wird zunächst während 2 Stunden bei Raumtemperatur und danach während 1 1/2 Stunden bei 55° gerührt. Anschliessend giesst man das Reaktionsgemisch auf Wasser und filtriert die gebildeten Kristalle ab. Umkristallisation des Rückstandes aus Acetonitril liefert reinen 1-(1-Methyl-4-oxo-2-imidazolin-2-yl)-3-(2-thienyl)-harnstoff, welcher unter Zersetzung bei 191-193° schmilzt.

Beispiel 2

Eine Suspension von 2,20 g (17,6 mMol) 3-Isocyanatothiophen und 2,03 g (17,6 mMol) Kreatinin in 20 ml wasserfreiem Dimethylformamid werden zunächst während 2 Stunden bei Raumtemperatur und danach während 3 Stunden bei 90° gerührt. Anschliessend giesst man das Reaktionsgemisch auf Wasser und filtriert die gebildeten Kristalle ab. Umkristallisation des Rückstandes aus Aceton liefert reinen 1-(1-Methyl-4-oxo-2-imidazolin-2-yl)-3-(3-thienyl)-harnstoff, welcher unter Zersetzung bei 199-201° schmilzt.

Das als Ausgangsmaterial verwendete 3-Isocyanatothiophen kann wie folgt hergestellt werden:

21,1 g (165 mMol) Thiophen-3-carbonsäure werden mit 36 ml (495 mMol) Thionylchlorid während einer Stunde zum Rückfluss erhitzt. Danach wird das überschüssige Thionylchlorid am Rotationsverdampfer unter vermindertem Druck entfernt. Destillation des Rückstandes am Wasserstrahlvakuum liefert Thiophen-3-carbonsäurechlorid, Sdp. 72-74°/14,3 mbar.

22,56 g (154 mMol) Thiophen-3-carbonsäurechlorid werden in 400 ml Aceton gelöst, auf -5° gekühlt und bei dieser Temperatur tropfenweise mit einer Lösung von 11,0 g (164 mMol) Natriumazid in 30 ml Wasser versetzt. Anschliessend rührt man das Reaktionsgemisch während einer Stunde bei 0°. Dann versetzt man das Reaktionsgemisch mit 1 l Wasser, extrahiert mehrmals mit Toluol und wäscht die vereinigten Toluolextrakte mit Wasser. Dann trocknet man die vereinigten Toluolextrakte über Magnesiumsulfat und erhitzt die so getrocknete Lösung während 2 Stunden zum Rückfluss. Danach wird das Lösungsmittel bei maximal 30° und einem Druck von 19,5 mbar am Rotationsverdampfer abgedampft. Destillation des Rückstandes am Wasserstrahlvakuum liefert 3-Isocyanatothiophen, Sdp. 45-46°/14,3 mbar.

## Beispiel 3

Eine Lösung von 15 g (80 mMol) 5-Chlorthiophen-3-carbonsäureazid in 155 ml abs. Dioxan wird unter Stickstoffatmosphäre 3 Stunden zum Rückfluss erhitzt. Zum Reaktionsgemisch, welches 5-Chlor-3-isocyanatothiophen enthält, gibt man dann 9,33 g (82,5 mMol) Kreatinin zu und rührt unter Stickstoffatmosphäre 3 Stunden bei 95°. Das Dioxan wird danach bis auf 20 ml abgedampft, und der Rückstand mit 150 ml Wasser versetzt und 30 Minuten bei Raumtemperatur gerührt. Das ausgefallene kristalline Produkt wird dann abfiltriert und zweimal aus Aceton unter Zusatz von Aktivkohle umkristallisiert, wobei man reinen 1-(5-Chlor-3-thienyl)-3-(1-methyl-4-oxo-2-imidazolin-2-yl)harnstoff erhält, welcher bei 190-191° schmilzt.

Das als Ausgangsmaterial verwendete 5-Chlorthiophen-3-carbonsäureazid kann wie folgt hergestellt werden:

15 g (92,3 mMol) 5-Chlorthiophen-3-carbonsäure werden in 70 ml abs. Aceton gelöst, mit 9,7 g (95,9mMol) Triäthylamin versetzt und auf 0° gekühlt. Dann werden 15 g (0,14 Mol) Chlorameisensäureäthylester zugetropft und noch 1/2 Stunde bei 0° gerührt. Danach werden ebenfalls bei 0° 9,3 g (0,14 Mol) Natriumazid in 24 ml Wasser zugetropft, und das Reaktionsgemisch noch 1 Stunde bei 0° weitergerührt. Das ausfallende Triäthylaminhydrochlorid wird abfiltriert und mit Aceton nachgewaschen. Das Filtrat wird bis zur Phasentrennung eingeengt. Danach wird die organische Phase abgetrennt, und die wässrige Phase noch dreimal mit je 100 ml Methylenchlorid extrahiert. Die vereinigten organischen Extrakte werden getrocknet und bei einer maximalen Badtemperatur von 30° zur Trockene eingedampft. Umkristallisation des Rückstandes aus n-Hexan liefert reines 5-Chlorthiophen-3-carbonsäureazid, welches bei 54° schmilzt.

Beispiel A

| Herstellung von Kapseln | pro Kapsel |
|---|---|
| 1-(1-Methyl-4-oxo-2-imidazolin-2-yl)-3-(3-thienyl)harnstoff | 10 mg |
| Milchzucker | 165 mg |
| Maisstärke | 30 mg |
| Talk | 5 mg |
| Kapselinhalt total | 210 mg |

Der Wirkstoff, der Milchzucker und die Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

## Beispiel B

| Herstellung von Tabletten | pro Tablette |
|---|---|
| 1-(1-Methyl-4-oxo-2-imidazolin-2-yl)-3-(3-thienyl)harnstoff | 10,0 mg |
| Milchzucker | 129,0 mg |
| Maisstärke | 50,0 mg |
| Gelatinierte Maisstärke | 8,0 mg |
| Calciumstearat | 3,0 mg |
| Totalgewicht | 200,0 mg |

Der Wirkstoff, der Milchzucker, die Maisstärke und die gelatinierte Maisstärke werden innig miteinander vermischt. Die Mischung wird durch eine Zerkleinerungsmaschine passiert und anschliessend mit Wasser zu einer dicken Paste befeuchtet. Die feuchte Masse wird durch ein Sieb passiert. Das feuchte Granulat wird bei 45° getrocknet. Das getrocknete Granulat wird mit dem Calciumstearat gründlich vermischt. Das Granulat wird nun zu Tabletten von einem Gewicht von 200 mg und einem Durchmesser von etwa 8 mm gepresst.

## Beispiel C

| Herstellung von Suppositorien | pro Supp. |
|---|---|
| 1-(1-Methyl-4-oxo-imidazolin-2-yl)-3-(3-thienyl)harnstoff | 0,010 g |
| Cacaobutter (Smp 36 bis 37°) | 1,245 g |
| Carnauba-Wachs | 0,045 g |
| Für ein Suppositorium von | 1,3 g |

Cacaobutter und Carnauba-Wachs werden in einem Glas- oder Stahlgefäss geschmolzen, gründlich vermengt und auf 45° abgekühlt. Hierauf gibt man den fein pulverisierten Wirkstoff zu und rührt, bis er vollständig dispergiert ist. Man giesst die Mischung in Suppositorienformen geeigneter Grösse, lässt abkühlen, nimmt dann die Suppositorien aus den Formen und verpackt sie einzeln in Wachspapier oder Metallfolien.

Beispiel D

| Herstellung von Tabletten | | pro Tablette |
|---|---|---|
| 1-(5-Chlor-3-thienyl)-3-(1-methyl-4-oxo-2-imidazolin-2-yl)harnstoff | | 2,0 mg |
| Milchzucker | | 100,0 mg |
| Maisstärke | | 67,5 mg |
| Magnesiumstearat | | 0,5 mg |
| | Totalgewicht | 170,0 mg |

Der Wirkstoff, der Milchzucker und ein Teil der Maisstärke werden gemischt, gesiebt, mit Maisstärke-Wasser-Kleister verarbeitet, granuliert, getrocknet und gesiebt. Das Granulat wird mit dem Magnesiumstearat gemischt und zu Tabletten à 170 mg und geeigneter Grösse verpresst.

Patentansprüche

1. Harnstoffderivate der allgemeinen Formel

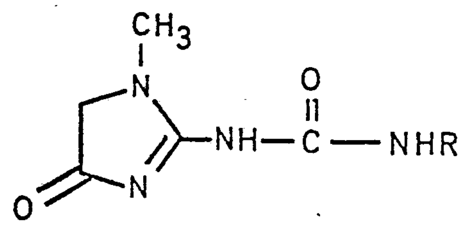

I

worin R 2-Thienyl, 3-Thienyl oder 5-Halogen-3-thienyl bedeutet.

2. Harnstoffderivate gemäss Anspruch 1, worin R 2-Thienyl oder 3-Thienyl bedeutet.

3. Harnstoffderivat gemäss Anspruch 1, worin R 5-Chlor-3-thienyl bedeutet.

4. Isocyanatothiophenderivate der allgemeinen Formel

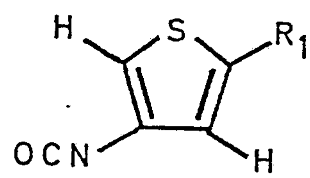

worin $R_1$ Wasserstoff oder Halogen bedeutet.

5. Harnstoffderivate gemäss einem der Ansprüche 1 bis 3 als pharmazeutische Wirkstoffe.

6. Harnstoffderivate gemäss einem der Ansprüche 1 bis 3 als Anxiolytika.

7. Verfahren zur Herstellung von Harnstoffderivaten gemäss einem der Ansprüche 1 bis 3, dadurch gekennzeichnet, dass man Kreatinin der Formel

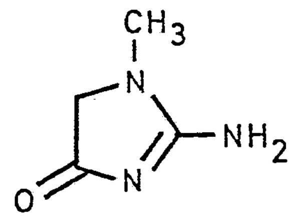

II

mit einem Isocyanatothiophen der allgemeinen Formel

$$OCN-R \qquad III$$

worin R 2-Thienyl, 3-Thienyl oder 5-Halogen-3-thienyl bedeutet,

umsetzt.

8. Arzneimittel enthaltend ein Harnstoffderivat gemäss einem der Ansprüche 1 bis 3.

9. Anxiolytikum enthaltend ein Harnstoffderivat gemäss einem der Ansprüche 1 bis 3.

10. Verwendung eines Harnstoffderivates gemäss einem der Ansprüche 1 bis 3 bei der Bekämpfung bzw. Verhütung von Krankheiten.

11. Verwendung eines Harnstoffderivates gemäss einem der Ansprüche 1 bis 3 bei der Bekämpfung von Angstzuständen.

***

Patentansprüche "für Oesterreich"

1. Verfahren zur Herstellung von Harnstoffderivaten der allgemeinen Formel

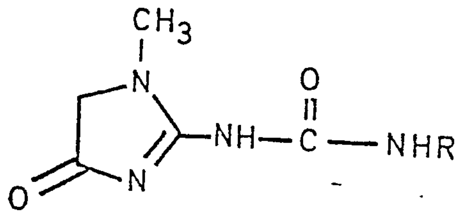

I

worin R 2-Thienyl, 3-Thienyl oder 5-Halogen-3-thienyl bedeutet,

dadurch gekennzeichnet, dass man Kreatinin der Formel

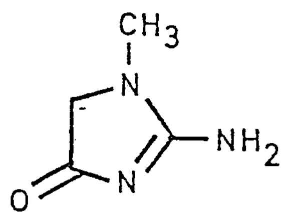

II

mit einem Isocyanatothiophen der allgemeinen Formel

OCN-R III

worin R die oben angegebene Bedeutung besitzt,

umsetzt.

2. Verfahren nach Anspruch 1, dadurch gekennzeichnet, dass man Kreatinin mit 2-Isocyanatothiophen oder 3-Isocyanatothiophen umsetzt.

3. Verfahren nach Anspruch 1, dadurch gekennzeichnet, dass man Kreatinin mit 5-Chlor-3-isocyanatothiophen umsetzt.

0016371

Europäisches Patentamt

# EUROPÄISCHER RECHERCHENBERICHT

Nummer der Anmeldung

EP 80 10 1097

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | DE - A1 - 2 448 869 (McNEIL LABORATORIES)<br>-- | | C 07 D 409/12<br>C 07 D 333/36<br>A 61 K 31/38<br>A 61 K 31/415 |
| A | US - A - 3 983 135 (McNEIL LABORATORIES)<br>-- | | |
| A | US - A - 4 025 517 (McNEIL LABORATORIES)<br>---- | | RECHERCHIERTE SACHGEBIETE (Int. Cl.3)<br>A 61 K 31/00<br>C 07 D 333/36<br>C 07 D 409/12 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 30-05-1980 | FROELICH |